(19) Europäisches Patentamt
European Patent Office
Office européen des brevets

(11) Veröffentlichungsnummer: **0 215 930**
**B1**

(12) **EUROPÄISCHE PATENTSCHRIFT**

(45) Veröffentlichungstag der Patentschrift: **27.06.90**

(51) Int. Cl.5: **C 01 B 3/34,** C 01 B 3/38, C 07 C 29/15

(21) Anmeldenummer: **86902305.1**

(22) Anmeldetag: **24.03.86**

(86) Internationale Anmeldenummer: **PCT/DE86/00129**

(87) Internationale Veröffentlichungsnummer: **WO 86/05769 09.10.86 Gazette 86/22**

# (54) ERZEUGUNG VON WÄRMEENERGIE DURCH VERBRENNUNG VON SYNTHESEGAS.

(30) Priorität: **25.03.85 DE 3511191**
**23.05.85 DE 3518467**
**17.07.85 DE 3525479**

(43) Veröffentlichungstag der Anmeldung: **01.04.87 Patentblatt 87/14**

(45) Bekanntmachung des Hinweises auf die Patenterteilung: **27.06.90 Patentblatt 90/26**

(84) Benannte Vertragsstaaten: **AT BE CH DE FR GB IT LI LU NL SE**

(56) Entgegenhaltungen:
**DE-A-1 645 851**
**DE-A-2 437 907**
**DE-A-2 618 961**
**DE-C- 306 301**
**FR-A-2 178 235**
**FR-A-2 339 587**
**FR-A-2 495 606**
**GB-A-1 006 745**

(73) Patentinhaber: **SCHICK, Josef Hubert**
**Kranichstrasse 1**
**D-5042 Erftstadt-Erp (DE)**

(72) Erfinder: **SCHICK, Josef Hubert**
**Kranichstrasse 1**
**D-5042 Erftstadt-Erp (DE)**

(74) Vertreter: **Berkenfeld, Helmut, Dipl.-Ing.**
**An der Schanz 2**
**D-5000 Köln 60 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 215 930 B1**

Courier Press, Leamington Spa, England.

**Beschreibung**

Die Erfindung betrifft ein Verfahren zum Erzeugen von Wärmeenergie aus Kohlenwasserstoffen (Primärbrennstoff) durch oxidative Umsetzung in einer Spaltungsreaktionsstufe und einer Verbrennungsreaktionsstufe, wobei primärer Brennstoff und aus der Verbrennungsreaktionsstufe stammendes Rauchgas in der Spaltungsreaktionsstufe zu Synthesegas umgesetzt werden.

Wasserdampf, kurz als "Dampf" bezeichnet, ist sowohl für die Umwandlung in elektrische Energie als auch für die Erzeugung von Wärme der am meisten benutzte und daher in relative großer Menge benötigte sekundäre Energieträger. Dampf wird bekanntermaßen in überwiegendem Maße durch die als Verbrennung bezeichnete oxidative Umsetzungsreaktion von fossilen Brennstoffen mit Sauerstoff, meist in Form von Luft, erzeugt, so zum Beispiel über Heizwerke und Heizkraftwerke zu Beheizungszwecken für insbesondere große Gebäudekomplexe, wie Verwaltungen, und auch für private Haushalte und anderes mehr.

Bei der üblichen Verbrennung von fossilen Brennstoffen wird der darin enthaltene Kohlenstoff mit Luftsauerstoff zu $CO_2$ (bzw. CO) oxidiert, und das entstandene Kohlenstoffoxidgas (Rauchgas) wird gewöhnlich einfach in die Atmosphäre abgegeben. Damit ist der wertvolle Rohstoff Kohlenstoff für die weitere Energiegewinnung verloren. Darüber hinaus werden bei der Verbrennung fossiler Brennstoffe — je nach Art des eingesetzten Brennstoffs — wichtige Beiprodukte, wie Schwefelverbindungen, Stickoxide und Blei, in die Atmosphäre abgegeben. Abgesehen davon, daß die Kohlenstoffreserven unserer Erde begrenzt sind und durch die bisherige Handhabung der Kohlenstoffoxid-Abgase der Kohlenstoff praktisch verschwendet wird, besteht, infolge der Belastung der Atmosphäre die Gefahr, daß, wenn diese Verfahrensweise beibehalten wird, unsere Umwelt in absehbarer Zeit völlig zerstört sein wird. Es kommt hinzu, daß Heizwerke möglichst nahe am Verbrauchergebiet liegen sollten, um durch zu lange Transporte entstehende Netzverluste zu vermeiden. Das setzt aber voraus, daß bei den in den Heizwerken praktizierten Verfahren zur Energiegewinnung möglichst keine umweltstörenden Abfallstoffe anfallen. Dieses Problem ist bisher nicht befriedigend gelöst.

Das erfindungsgemäße Verfahren läßt eine hohe Wirtschaftlichkeit zu. Die kohlenstoffhaltigen Verbrennungsprodukte werden so im Kreislauf geführt, daß sie als wiederverwendbare sekundäre Brennstoffe und/oder chemische Rohstoffe voll verwertet werden. Das Verfahren ermöglicht so ein vollständiges Kohlenstoffrecycling und vermeidet das Abblasen von Rauchgasen in die Atmosphäre. Es fallen keinerlei die Umwelt störende Abfallstoffe an, so daß das Verfahren in nächster Nähe von Siedlungsgebieten betrieben werden und somit kostengünstig nahe am Verbrauchsgebiet zwecks Gewinnung von Wärmeenergie arbeiten kann.

Als beim erfindungsgemäßen Verfahren eingesetzte Ausgangs-Brennstoffe, die im folgenden "primärer Brennstoff" genannt werden, können beliebige bekannte reduzierend wirkende Kohlenwasserstoff-Verbindungen und deren Gemische dienen. So kann man beispielsweise als primären Brennstoff Methan, Erdgas, Kohlegas, Spaltgas, Synthesegas, Stadtgas, Kokereigas sowie Erdölprodukte, wie Naphthene und dergleichen, und auch reduzierende Kohlenwasserstoffe enthaltende Abgase aus chemischen Prozessen einsetzen. Bevorzugt wird Methan verwendet. Man kann andere einzusetzende Gase oder Gasgemische gewünschtenfalls auch zunächst einer Methanisierung unterwerfen und das methanisierte Produkt als primären Brennstoff beim erfindungsgemäßen Verfahren einsetzen.

Bekannt ist ein Verfahren (DE—A 1 645 851) zum Herstellen von Methanolsynthesegas. Dieses Verfahren betrifft eine Verbesserung der bis dahin bekannten Verfahren zur Methanolsynthese. Bei diesen bekannten Verfahren wird Methan mit $CO_2$ und Wasserdampf zu CO und $H_2$ (Synthesegas) umgesetzt und dieses katalytisch zu Methanol weiterreagiert. Die in DE—A 1 645 851 beschriebene Verbesserung besteht darin, das benötigte $CO_2$ aus den Rauchgasen der Heizung des Spaltofens zu gewinnen. Der Spaltofen wird mit einem Gemisch aus Methan und Luft geheizt, so daß ein Rauchgas der ungefähren Zusammensetzung $CO_2$ (8%), $O_2$ (1,7%), $N_2$ (72%) und $H_2O$ (18,5%) entsteht (Seite 10, Absatz 1). Aus diesem soll durch Auswaschen mit Aminosäuresalzen das $CO_2$ isoliert und durch Erhitzen wieder freigesetzt und für die Synthesegasherstellung zur Verfügung gestellt werden. Da dabei der noch enthaltene Restsauerstoff (die 1,7%) stört, soll dieser nach der Erfindung der Entgegenhaltung mit Entspannungsgas ($CO_2$/CO/$H_2$/$CH_4$-Gemisch gemäß Seite 9, Absatz 3) und Dimethyläther katalytisch verbrannt und entfernt werden. 50% des so erhaltenen Rauchgases werden mit einer Temperatur von 150°C in die Atmosphäre abgegeben. Das enthaltene $CO_2$ und die Energie gehen daher verloren. Diese Abgabe von $CO_2$ in die Atmosphäre und der sich dadurch ergebende Verlust spielten bei dem in DE—A 1 645 851 beschriebenen Verfahren jedoch keine Rolle, da mit diesem ausschließlich die Methanolsynthese verbessert werden soll.

Aufgabe der Erfindung ist, Dampf als Wärmeträger und damit Wärme und Strom so zu erzeugen, daß hierbei kein Verlust an Kohlenstoff entsteht und keine Schadstoffe an die Umwelt abgegeben werden. Zusätzlich sollen zur Wärmegewinnung nicht benötigte Kohlenwasserstoffverbindungen als Ausgangsstoffe für andere chemische Prozesse gewonnen werden.

Die Lösung für diese Aufgabe ergibt sich bei einem Verfahren der eingangs genannten Gattung nach der Erfindung dadurch, daß das Synthesegas aus der Spaltungsreaktionsstufe bzw. eine daraus durch katalytische Umsetzung in einer zusätzlichen Katalysestufe gebildete sauerstoffhaltige Kohlenstoffverbindung als sekundärer Brennstoff mit Sauerstoff in der Verbrennungsreaktionsstufe unter Gewinnung von Wärmeenergie in Form von Dampf oxidativ verbrannt und sämtliches bei der Verbrennung

gebildetes Rauchgas zurückgeführt wird und/oder für die Bildung von Kohlenstoffoxid im Kreislauf nicht benötigter sekundärer Brennstoff ausgeschleust wird. Die Heizung des Spaltofens wird somit nicht mit Methan(Primärbrennstoff)/Luft durchgeführt, sondern ein Teil des aus dem Spaltprozeß stammenden Synthesegases ($CO_2/CO/H_2$) (Sekundärbrennstoffe) wird vorzugsweise mit Sauerstoff verbrannt, so daß das entstehende Gemisch ($CO_2/CO/H_2O$) direkt wieder komprimiert und mit zusätzlichem Primärbrennstoff dem Spaltprozeß zugeführt werden kann. Geringe Spuren von $O_2$, die noch enthalten sind, stören dabei nicht. Dieser stark exotherme Prozeß, bei dem wie gesagt keine Abgase entstehen, dient der Erzeugung von Wärmeenergie, die über Dampfkessel oder Entspannungsturbinen genutzt wird. Es fällt Synthesegas an, welches katalytisch in nützliche Produkte wie Methanol, Ethanol, Acetaldehyd usw. umgesetzt werden kann, wobei zusätzliche Wärmeenergie entsteht und genutzt werden kann. Alternativ wird das gesamte im Spaltprozeß gebildete Synthesegas zunächst zu Methanol umgesetzt und ein Teil desselben als alterniver "Sekundärbrennstoff" verbrannt, um einerseits Energie zu gewinnen und andererseits ein $CO_2$—, CO— und $H_2O$-haltiges Gemisch zu erzeugen, welches zur $CO_2$-Anreicherung dem Primärbrennstoff zugemischt wird, um wiederum im Spaltprozeß im Primär-Reformer Synthesegas zu erzeugen. Auch hierbei wird das gesamte gebildete $CO_2$ im Kreislauf geführt, so daß keine Abgase entstehen.

In den Unteransprüchen werden vorteilhafte und förderliche Weiterbildungen angegeben.

Die primären Brennstoffe werden beim erfindungsgemäßen Verfahren zweckmäßig gasförmig eingesetzt. Es ist bei Verwendung von zum Beispiel höheren Kohlenwasserstoffen, wie Paraffinen oder Erdöl, Olefinen oder Naphthenen, daher vorteilhaft, daß man diese vor Einführung in das Verfahren in den gasförmigen Zustand bringt.

Es versteht sich, daß man auch von beliebigen festen fossilen Brennstoffen, wie zum Beispiel Braunkohle, Steinkohle oder dergleichen als Rohstoff ausgehen kann. Diese muß man dann aber gemäß einer der bekannten Vergasungsreaktionen (Methanbildungsreaktionen) zunächst zu gasförmigen, reduzierend wirkenden Kohlenwasserstoffen aufarbeiten.

In der Spaltungsreaktionsstufe des erfindungsgamäßen Verfahrens wird der primäre Brennstoff zusammen mit dem Rauchgas, das aus der Verbrennungsreaktionsstufe des erfindungsgemäßen Verfahrens stammt, zu Synthesegas umgesetzt. Soweit hierbei auch noch $CO_2$ entsteht, wird dieses aus dem Gasgemisch ausgewaschen und im Kreislauf in die Spaltungsreaktionsstufe wieder eingeführt oder — gewünschtenfalls — in der nachgeschalteten Katalysereaktionsstufe zugesetzt. In der Spaltungsreaktionsstufe werden Methan und Kohlensäure in im wesentlichen Kohlenmonoxid und Wasserstoff gespalten. Bei dieser Spaltungsreaktion können der primäre Brennstoff und das Kohlenstoffoxidgas thermisch umgesetzt werden, wobei die Reduktion endotherm oder autotherm und zweckmäßig katalytisch geführt wird. Bei der katalytischen Umsetzung verwendet man vorteilhaft einen nickelhaltigen oder einen mangesiumhaltigen Katalysator. Es können dabei die folgenden Reaktionen ablaufen:

$$CH_4 + CO_2 \rightarrow 2\,CO + 2\,H_2$$

$$CH_4 + CO_2 \xrightarrow{Ni} 2\,CO + 2\,H_2$$

$$CH_4 + H_2O \xrightarrow{Ni} CO + 3\,H_2$$

$$3\,CH_4 + CO_2 + 2H_2O \rightarrow 4\,CO + 8\,H_2.$$

Reaktionsführung in der Spaltungsreaktionsstufe und Zusammensetzung des darinresultierenden Synthesegases lassen sich mit einer gewissen Variationsbreite steuern.

So läßt sich beispielsweise die Zusammensetzung des dem primären Brennstoff zugeführten Rauchgases durch das der Verbrennungsreaktionsstufe zuzuführende Oxidationsmittel variieren. Wenn man, was vorteilhaft sein kann, als Oxidationsmittel in der Verbrennungsreaktionsstufe reines Sauerstoffgas benutzt, gewinnt man als Abgas aus der Verbrennungsreaktionsstufe stickstofffreies Rauchgas. Dieses enthält zwar eine gewisse Menge an Wasserdampf. Dieser stört, wie die vorstehenden chemischen Reaktionsformeln zeigen, in der Spaltungsreaktionsstufe nicht. Es ist daher möglich, in diesem Fall das Abgas aus der Verbrennungsreaktionsstufe ohne zusätzliche Bearbeitung, wie Kühlung oder Reinigung, der Spaltungsreaktionsstufe zuzuleiten. Meist wird es sich jedoch als energetisch günstiger erweisen, wenn man das aus der Verbrennungsreaktionsstufe abgezogene Abgas zunächst einer Kühlung unterwirft und dabei den darin enthaltenen Wasseranteil vollständig oder weitgehend abtrennt. Das hat den Vorteil, daß man als Kühlmittel für das Abgas den primären Brennstoff vor der Einleitung in die Spaltungsreaktionsstufe benutzen und diesen vorteilhaft vorwärmen kann. Das in dieser Kühlstufe aus dem Abgas abgeschiedene Wasser wird beispielsweise demjenigen Wasservorrat zugeführt, der die in der Verbrennungsreaktionsstufe freiwerdende Wärme in Form von Wasserdampf als Wärmeträger aufnimmt.

Das $CO:H_2$-Verhältnis des in der Spaltungsreaktionsstufe gebildete Synthesegases kann unterschiedlich sein. Dieses Verhältnis wird an das in der nachfolgenden Katalysereaktionsstufe angestrebte sauerstoffhaltige Kohlenstoff-Produkt angepaßt. Soll beispielsweise Alkohol, zum Beispiel

Methanol, in der Katalysereaktionsstufe als sauerstoffhaltiges Kohlenstoffprodukt, im nachfolgenden "sekundärer Brennstoff" genannt, gewonnen werden, stellt man das Synthesegas aus der Spaltungsreaktionsstufe zweckmäßig auf ein $CO:H_2$-Verhältnis von 1:2, vorteilhaft 1:2,2, ein. Sollten beispielsweise Isobutylöl-Verbindungen als sekundärer Brennstoff in der Katalysereaktionsstufe gebildet werden, wird das Synthesegas in der Spaltungsreaktionsstufe mit einem entsprechend höheren CO-Gehalt eingestellt.

Man kann zur Regulierung des $CO:H_2$-Verhältnisses zusätzlich Wasserstoff in die Spaltungsreaktionsstufe einleiten. Man kann in der Spaltungsreaktionsstufe zur Vermeidung von Rußbildung auch mit Dampf arbeiten.

Wenn das Synthesegas mit hoher Temperatur die Spaltungsreaktionsstufe verläßt, kann man es zur Nutzung der Restenthalpie durch eine Enspannungseinrichtung leiten, bevor man es der Katalysereaktionsstufe zuführt.

Die Art des sekundären Brennstoffes, der in der Katalysereaktionsstufe gebildet wird, bestimmt sich von Fall zu Fall je nach Bedarf an chemischen Rohstoff als Produkt-Substanz. Es ist zweckmäßig, beim erfindungsgemäßen Verfahren einen Teil des sekundären Brennstoffes zur direkten Verwendung (Produkt-Substanz) als chemischer Rohstoff aus dem Kreisporzeß abzuziehen und nur den restlichen Teil des sekundären Brennstoffes aus der Katalysereaktionsstufe in die Verbrennungsreaktionsstufe zu führen. Vorteilhaft ist es, wenn man eine solche Menge an sekundärem Brennstoff als Produkt-Substanz aus dem erfindungsgemäßen Kreisprozeß abzieht und als chemischen Rohstoff direkt werwendet, die etwa äquivalent ist der Menge der an der Spaltungsreaktionsstufe frisch zugeführten primären Brennstoffes. Dies hat den Vorteil, daß die menge an Kohlenstoff-Verbindung, die im Kreislauf geführt wird, etwa gleich bleibt. Die im Kreislauf geführte Kohlenstoffmenge bleibt konstant.

Beim erfindungsgemäßen Verfahren ist es auch möglich, das Synthesegas aus der Spaltungsreaktionsstufe nur zum Teil in die Katalysereaktionsstufe zu führen und zum anderen Teil direkt in die Verbrennungsreaktionsstufe einzuleiten. Wahlweise kann man das Synthesegas aus der Spaltungsreaktionsstufe zu einem Teil in die Katalysereaktionsstufe und zu einem anderen Teil in eine nebengeschaltete gesonderte Umsetzungsstufe führen. Dies empfiehlt sich, wenn Rohstoffe als Beiprodukte gewonnen werden sollen.

Aus der Spaltungsreaktionsstufe abgezogenes Synthesegas läßt sich mit anderer Stelle des Kreisprozesses abgezogener Substanz umsetzen. Als Beispiel sei eine aus der Katalysereaktionsstufe entnommene sauerstoffhaltige Kohlenstoffverbindung genannt. Letztere kann man auch mit dem dem Synthesegas entnommen Kohlenmonoxid umgesetzt werden. Dabei werden in der chemischen Industrie so wichtige chemische Rohstoffe, wie Acetaldehyd und Ethanol als Beiprodukte des erfindungsgemäßen Verfahrens nach den folgenden theoretischen Reaktionen gewonnen:

$2\,CO + 2\,H_2 \rightarrow CO + CH_3OH$ (sekundärer Brennstoff)
$CO + CH_4$ (primärer Brennstoff) $\rightarrow CH_3CHO$ (Acetaldehyd)
$CH_3CHO + H_2 \rightarrow C_2H_5OH$ (Ethanol)

In der Katalysereaktionsstufe wird das Synthesegas zweckmäßig exotherm in Anwesenheit eines kupferhaltigen Katalysators umgesetzt. Die Reaktion läuft dabei theoretisch nach der folgenden Gleichung ab:

$$2\,CO + 4\,H_2 \rightarrow 2\,CH_3OH$$

Wenn das aus der Spaltungsreaktionsstufe abgezogene Synthesegas ein höheres Verhältnis an $CO:H_2$ als 1:2 hat, empfiehlt es sich, der Katalysereaktionsstufe zusätzlich Wasserstoff zuzuleiten. Wenn zur Vermeidung von Wachsbildung und Bildung von höheren Kohlenwasserstoffen die Anwesenheit von $CH_4$ erforderlich ist, kann dieses, abgezweigt von der Eintrittsleitung für den primären Brennstoff, zusätzlich in die Katalysereaktionsstufe eingeleitet werden. Es ergeben sich praktisch die folgenden Reaktionsmechanismen:

1. $CO + 2H_2 \rightleftarrows CH_3OH$
2. $CO_2 + 3H_2 \rightleftarrows CH_3OH + H_2O$
3. $CH_4 + H_2O \rightleftarrows 3H_2 + CO$
4. $CO + H_2O \rightleftarrows H_2 + CO_2$

Die Gleichgewichtsreaktion zugunsen der Methanolbildung ist abhängig von der Stöchiometriezahl:

$$(H_2 - CO_2)/(CO + CO_2)$$

Dieses Verhältnis wird bestimmt durch das $CH_4/CO_2$-Verhältnis am Eintritt in die Spaltungsreaktionsstufe.

Die Wärme, die bei einer exothermen Reaktion in der Katalysereaktionsstufe frei wird, kann man vorteilhaft durch Dampf als Wärmeträger gewinnen und beliebig nutzen.

In der Verbrennungsreaktionsstufe wird dersekundäre Brennstoff mittels des dieser Verbrennungsreaktionsstufe zugeführten Oxidationsmittel, vorzugsweise reiner Sauerstoff oder auch Luft, verbrannt. Die dabei gewonnene Wärmeenergie wird mit Wasserdampf als Wärmeträger zum Energieverbraucher abgeführt. Das Abgas wird gekühlt oder ungekühlt zusammen mit frischem primärem Brennstoff wieder in die Spaltungsreaktionsstufe geführt und darin wie zuvor beschrieben weiterverwendet.

Wenn Luft als Oxidationsmittel in der Verbrennungsreaktionsstufe benutzt wird, ist es zweckmäßig, vor dem Zusammenbringen des Abgases mit dem frischen primären Brennstoff aus dem Abgas die kohlenstofffreien Bestandteile abzutrennen. Den dabie abgetrennten Stickstoff kann man in die Atmosphäre abblasen. Dabei geht Brennstoff nicht verloren und die Atmosphäre verschmutzt nicht. Es handelt sich bei diesem Stickstoff um das praktisch einzige Abfallprodukt aus dem erfindungsgemäßen Kreisprozeß.

Dieser Stickstoff fällt in relative reiner Form an. Deshalb kann man ihn nutzbringend verwenden. Beispielsweise dient er als Kühlmittel oder Inertgas, falls dies standortbedingt wirtschaftlich vernünftig ist.

Die Trennung von kohlenstoffhaltigen und kohlenstofffreien Bestandteilen in dem Abgas aus der Verbrennungsreaktionsstufe erfolgt vorteilhaft in einer Gaszerlegungsstufe, die der Verbrennungsreaktionsstufe nachgeschaltet wird. Mam kann in dieser Gaszerlegungsstufe zum Beispiel mittels eines Druck-Temperaturverfahrens, einer Waschflüssigkeit, eines kombinierten Druck-Temperatur-Waschverfahrens, auch mittels semipermeabler Membran oder mittels eines Fremdgasdiffusionsverfahrens das Abgas zerlegen.

Am Beispiel der in der Zeichnung schematisch dargestellten Fließbilder wird die Erfindung nun weiter erläutert. In der Zeichnung ist:

Fig. 1 ein Fließbild mit der Darstellung des grundsätzlichen Verfahrensablaufs,

Fig. 2 ein Fließbild mit der Darstellung eines Verfahrensablaufes mit zusätzlichen Stufen,

Fig. 3 ein Fließbild mit der Darstellung eines abgewandelten Verfahrensablaufs,

Fig. 4 ein Fließbild mit der Darstellung eines weiter abgewandelten Verfahrensablaufs,

Fig. 5 ein Fließbild mit der Darstellung eines weiter abgewandelten Verfahrensablaufs,

Fig. 6 ein Fließbild mit der Darstellung eines weiter abgewandelten Verfahrensablaufs mit zusätzlichen Reaktionsstufen zum Gewinnen von Produkt-Substanzen,

Fig. 7 ein Fließbild mit der Darstellung eines Verfahrensablaufs, bei dem Methan als primärer Brennstoff eingeleitet wird,

Fig. 8 ein Fließbild mit der Darstellung eines Verfahrensablaufs ähnlich dem nach Fig. 7, bei dem das Synthesegas mit Luft verbrannt wird,

Fig. 9 ein Fließbild mit der Darstellung eines Verfahrensablaufs, bei dem Methan und Wasserstoff als primärer Brennstoff verwendet werden,

Fig. 10 ein Fließbild mit der Darstellung eines weiter abgewandelten Verfahrensablaufs und

Fig. 11 ein Fließbild mit der Darstellung eines Verfahrensablaufs, bei dem ohne Katalysereaktionsstufe gefahren wird.

Bei allen Ausführungsformen wird als primärer Brennstoff $CH_4$ durch eine Leitung 2 in den Kreisprozeß eingeleitet.

Gemäß der Darstellung in Fig. 1 wird der primäre Brennstoff durch die Leitung 2 direkt in die Spaltungsreaktionsstufe 17 eingeführt, in die gleichzeitig durch die Leitung 3 die Rauchgase gelangen, die aus der Verbrennungsreaktionsstufe 11 über die Leitung 9 zuströmen. In der Spaltungsreaktionsstufe 17 werden der primäre Brennstoff und das Rauchgas in Kohlenmonoxid und Wasserstoff gespalten. Das dabei gebildete Synthesegas wird durch Leitung 4 in die Katalysereaktionsstufe 18 geführt und in dieser zu dem sekundären Brennstoff $CH_3OH$ umgewandelt. Diese Reaktion verläuft exotherm. Man kann die hierbei freiwerdende Wärme gegen siedendes Wasser isotherm wiedergewinnen. Diese Wärmeenergie steht als Dampf zur Verfügung.

Aus der Spaltungsreaktionsstufe tritt das Synthesegas mit einem hohem Dampfanteil und dem sogenannten $CO_2$-Kreislaufgas aus. Die Temperatur beträgt 860°C. Der Druck betragt 15 bar. Der hohe Energiegehalt dieses Gas-Dampfgemisches wird genutzt. Hierzu wird es einem Abhitzekessel zugeführt. In diesem wird diejenige Dampfmenge erzeugt, die für den Spaltprozeß im Reaktor 17, dem Primär-Reformer, erforderlich ist. Das Gas-Dampfgemisch verläßt das Abhitzesystem mit einer Temperatur von ca. 250°C, einem Druck von 14,5 bar und besitzt damit immer noch eine hohe Energie. Auch diese wird genutzt. Das Dampf-Gasgemisch wird einer Entspannungsturbine zugeleitet. Diese treibt eine Verdichtergruppe an. Diese verdichtet

1. das aus der $CO_2$-Wäsche austretende Synthesegas für die nachfolgende Methanol-Synthese,
2. das Kreislauf-$CO_2$ und
3. die durch die Leitungen 2 und 3 zuzuführenden und zu spaltenden Gase ($CH_4 + CO_2$).

Die Austrittstemperatur des Gas-Dampfgemisches hängt von der gesamten Verdichterleistung einer Anlage ab. Im Prinzip kann die Energie des Gas-Dampfgemisches am Eintritt der Entspannungsturbine bis zur Kondensation des in dem Gas-Dampfgemisches enthaltenden Wasserdampfanteils ausgenutzt werden. In diesem Fall handlet es sich bei der Antriebsturbine um eine sogenannte Kondensationsturbine und wenn

wegen der Mengenverhältnisse der zu verdichtenden Gase nur wenig Energie benötigt, wird kann die überschüssige Energie für eine zusätzliche Dampferzeugung im Abhitzesystem genutzt werden.

Der sekundäre Brennstoff, das $CH_3OH$, wird durch Leitung 6 in den Kessel für die Verbrennungsreaktionsstufe 11 geführt. In diesem wird er mit dem durch die Leitung 7 zugesetzten Oxidationsmittel verbrannt. Die dabei freiwerdende Wärmeenergie wird mit dem durch die Leitung 8 geschickten Wassers mit Wasserdampf als Wärmeträger gewonnen und steht zur beliebigen weiteren Verwendung zur Verfügung.

Bei der in Figur 2 dargestellten Ausführungsform wird in der Verbrennungsreaktionsstufe 11 als Oxidationsmittel durch die Leitung 7 Luft eingebracht. Das durch die Leitung 9 daraus abgezogene Abgas wird zunächst in einem Kühler 12 vorgekühlt. Als Kühlmittel dient der durch die Leitung 2 eingeführte primäre Brennstoff. Dieser wird dadurch vorgewärmt und im vorgewärmeten Zustand dem Reaktor 17, der Spaltungsreaktionsstufe, zugeführt. Das in dem Kühler 12 gekühlte Abgas wird über die Leitung 9a weitergeleitet, in einem Verdichter 13 verdichtet und daraus durch die Leitung 9b einer Kolonne 14 zugeführt. In dieser werden die kohlenstoffhaltigen Gasbestandteile von den Stickstoff-Gasbestandteilen getrennt. Am Kopf der Kolonne 14 wird durch die Leitung 10a Stickstoff abgezogen, der in der Entspannungseinrichtung 16, beispielsweise einer Entspannungsturbine, entspannt und dann durch die Leitung 10 an die Atmosphäre abgegeben oder zur weiteren sonstigen Verwendung entnommen werden kann.

Das stickstofffreie Rauchgas wird aus der Kolonne 14 durch die Leitung 9c einem Verdampfer 15 zugeführt. Von dort gelangt es durch die Leitung 3 in den Reaktor 17. Das in der Spaltungsreaktionsstufe im Reaktor 17 gebildete Synthesegas wird durch die Leitung 4 in die Katalysreaktionsstufe 18 geführt. Das darin gebildete Methanol wird zum Teil durch die Leitung 5 als Produkt-Methanol, als chemischer Rohstoff für gesonderte Umsetzung, abgezogen. Zum Teil wird es als sekundärer Brennstoff durch die Leitung 6 der Verbrennungsreaktionsstufe im Kessel 11 zugeführt und darin verbrannt. Dabei wird wieder die gewonnene Verbrennungsenergie mit dem durch die Leitung 8 zugeführten Wasserdampf als Wärmeträger zur beliebigen Nutzung abgeführt. Gewünschtenfalls kann das durch die Leitung 5 aus der Katalysereaktionsstufe abgezogene Produkt-Methanol, das infolge der exothermen Reaktion in der Katalysereaktionsstufe 18 einen gewissen Wärmegehalt besitzt, als Heizmittel für die Entspannungsstufe im Verdampfer 15 dienen, ehe es zur beliebigen sonstigen Verwendung als chemischer Rohstoff aus dem Kresiprozeß abgezogen wird.

Die in Figur 3 dargestellte Ausführungsform ist eine Abwandlung des Verfahrens gemäß der Figur 1. Es wird das Abgas, das durch die Leitung 9 aus der Verbrennungsreaktionsstufe 11 abgezogen wird, zunachst einem Kühler 12 zugeführt und darin vorgekühlt, ehe es durch die Leitung 3 der Spaltungsreaktionsstufe im Reaktor 17 zugeführt wird. Als Kühlmittel in dem Kühler 12 dient der durch die Leitung 2 in den Kreisprozeß eingebrachte primäre Brennstoff, der in dieser Weise vorgewärmt ebenfalls dem Reaktor 17 zugeführt wird. Aus dem Kühler 12 wird bei der Kühlung sich abscheidendes Wasser, das im Abgas enthalten ist, durch eine Leitung 29 abgezogen. Man kann dieses Wasser dem als Wärmeträger in der Leitung 8 durch den Verbrennungsreaktionskessel 11 geführten Wasser zusetzen.

Die Verfahrensführung zwischen der Spaltungsreaktionsstufe 17, der Katalysereaktionsstufe 18 und der Verbrennungsreaktionsstufe 11 ist bei dieser Ausführungsform die gleiche, wie sie zuvor im Zusammenhang mit Figur 2 beschrieben wurde. Der Unterschied besteht darin, daß das durch die Leitung 7 in die Verbrennungsreaktionsstufe 11 eingeführte Oxidationsmittel reiner Sauerstoff ist, der in einer vorgeschalteten Luftzerlegungsanlage 26 aus der durch Leitung 27 eingeleiteten Luft gewonnen wird. Auch in diesem Fall ist Stickstoff wieder das einzige neben dem Kondenswasser aus dem Kühler 12 anfallende Abfallprodukt. Allerdings wird der Stickstoff bei dieser Verfahrensführung durch die Leitung 28 der Luftzerlegungseinrichtung 26 abgezogen. Er kann, wie zuvor im Zusammengang mit Figur 2 beschrieben, behandelt bzw. verwendet werden.

Eine weitere Ausführungsform zeigt Figur 4. Die Verfahrensführung hinsichtlich der Verbrennungsreaktionsstufe 11, der Behandlung des daraus entweichenden Abgases und der Beschickung der Spaltungsreaktionsstufe 17 sind wie im Zusammenhang mit Figur 3 beschrieben wurde. Unterschiedlich ist, daß das aus der Spaltungsreaktionsstufe 17 durch die Leitung 4 abgezogene Synthesegas vor Einleitung indie Katalysereaktionsstufe 18 geteilt wird. Ein Teil wird durch die Leitung 4a der Katalysereaktionsstufe 18 zugeleitet und dort, wie beschrieben, indie sauerstoffhaltige Kohlenstoffverbindung $CH_3OH$ umgesetzt, die über die Leitung 5 als Produkt-Methanol gewonnen wird. Der andere Teil des Synthesegases wird durch die Leitung 4b direkt als sekundärer Brennstoff in die Verbrennungsreaktionsstufe 11 eingeleitet und dort verbrannt. Die Energiegewinnung erfolgt über den inder Leitung 8 geführten Wärmeträger.

Die Ausfuhrungsform nach Figur 5 ähnelt der in Zusammenhang mit Figur 2 beschriebenen Verfahrensführung. Bei dieser Ausführungsform sind jedoch zwei Kühler 12a und 12b zwischen der Verbrennungsreaktionsstufe 11 und der Gaszerlegungsstufe, die aus dem Verdichter 13, der Kolonne 14 und den Entspannungseinrichtungen 15 und 16 besteht, vorhanden. In dem Kühler 12a wird das Abgas in einer ersten Kühlstufe vorgekühlt. Als Kühlmittel wird durch die Leitung 7 Luft geschickt, die in der Zuleitung 6, in der der sekundäre Brennstoff herangeführt wird, weitergeleitet wird. In diesem Fall findet somit schon vor Eintritt in die Verbrennungsreaktionsstufe 11 ein Vermischen des Oxidationsmittels Luft mit dem sekundären Brennstoff statt. Dies wirkt sich auf den Wirkungsgrad der Verbrennungsreaktion

vorteilhaft aus. Das in dem ersten Kühler 12a vorgekühlte Abgas wird durch die Leitung 9d dem zweiten Kühler 12b zugeführt. In diesem wird es mit dem durch die Leitung 2 geschickten primären Brennstoff als Kühlmittel gekühlt. Von dort erfolgt die Verfahrensführung und die Weiterverarbeitung des Abgases in der gleichen Weise wie im Zusammenhang mit Figur 2 bechrieben. Aus dem Kühler 12b wird das kondensierte Wasser über die Leitung 29 abgezogen und teilweise bei 30 aus dem Kreisprozeß entfernt. Teilweise geschieht dies über die Leitung 31. Aus dieser wird es als Wärmeträger zur Energieaufnahme der Leitung 8 zugeführt.

Die Figur 6 zeigt in ihrer ausgezogenen Darstellung die Verfahrensführung gemäß Figur 2. Gestrichelt wird eine Variante zur Gewinnung zusätzlicher als chemische Rohstoffe einsatzfähiger Produkt-Substanzen gezeigt. Bei dieser Ausführungsform wird nur ein Teil des in dem Reaktor 17 gebildeten Synthesegases durch die Leitung 4 der Katalysereaktionsstufe 18 zugeführt. Ein anderer Teil wird durch die Leitung 23 in einen nebengeschalteten Umsetzungsreaktor 21 geführt. In diesem wird das Synthesegas gemäß der Gleichung

$$2\,CO + 2\,H_2 \rightarrow CH_3OH + CO$$

umgesetzt. Das CO wird dem Umsetzungsreaktor 21 entnommen und durch Leitung 24 einem weiteren Umsetzungsreaktor 19 zugeführt. Abgezweigt von der Leitung 2 wird diesem durch eine Leitung 2a Methan eingeführt. In dem Umsetzungsreaktor 19 wird gemäß der Gleichung

$$CH_4 + CO \rightarrow CH_3CHO$$

Acetaldehyd gewonnen. Dieser wird durch eine Leitung 25 in eine weitere Umsetzungsstufe, einen Katalysatorreaktor 20, überführt. In diesem wird er mit dem über die Zuleitung 32 zugesetzten Wasserstoff gemäß der Reaktionsformel

$$CH_3\,CHO + H_2 \rightarrow C_2H_5OH$$

zu Ethanol reduziert. Dieser Alkohol wird durch die Leitung 22 als Produkt-Ethanol entnommen.

Das in dem Umsetzungsreaktor 21 gebildete $CH_3OH$ wird als zusätzlicher sekundärer Brennstoff aus dem Reaktor 21 durch die Leitung 6a der Leitung 6 zugeführt. Auf diesem Weg gelangt er zusammen mit aus der Katalysereaktionsstufe 18 stammenden sekundärem Brennstoff in die Verbrennungsreaktionsstufe 11. Bei dieser Ausführungsform fallen zusätzlich zu er über den Wärmeträger 8 abgezogenen Wärmeenergie und dem aus der Leitung 10 bzw. 28 abgezogen Stickstoff sowie dem aus der Leitung 29 bzw. 30 abgezogen Wasser Produkt-Nethanol und Produkt-Ethanol an. Das erstere wird über die Leitung 5 und das letztere über die Leitung 22 abgezogen. Wahlweise kann man auch aus dem Umsetzungsreaktor 19 Acetaldehyd als weiterverwendbares Produkt entnehmen.

In Figur 7 ist ein spezielles Ausführungsbeispiel veranschaulicht. Methan wird als primärer Brennstoff in den Kreislaufprozeß eingeleitet und im Mischer mit dem aus der Verbrennungsreaktionsstufe im Kessel 11 stammenden Rauchgas vermischt. Das Gemisch wird im Verdichter komprimiert und in der Spaltungsreaktionsstufe zu Synthesegas umgesetzt. Aus der Spaltungsreaktionsstufe tritt das Synthesegas mit einem hohen Dampfantieil und dem sogenannten $CO_2$-Kreislaufgas aus. Die Temperatur beträgt 860°C, bei einem Druck von 15 bar. Der hohe Energiegehalt dieses Gas-Dampfgemisches wird zunächst dadurch genutzt, daß das Gas-Dampfgemisch einem Abhitzekessel zugeführt wird, in welchem die Dampfmenge produziert wird, die für den Spaltprozeß im Reaktor 17, dem Primär-Reformer, erforderlich ist. Das Gas-Dampfgemisch verläßt das Abhitzesystem mit einer Temperatur von ca. 250°C und 14.5 bar Druck und besitzt somit immer noch eine hohe Energie. Diese Energie wird nun dadurch verwertet, daß das Dampf-Gasgemisch einer Entspannungsturbine zugeleitet wird, die als Antrieb für die Verdichtergruppe dient. Mittels dieser Verdichtergruppe wird

1. das aus der $CO_2$-Wäsche austretende Synthesegas für die nachfolgenden Methanolsynthese verdichtet,
2. das Kreislauf-$CO_2$ verdichtet, und
3. auch die Kompression der durch die Leitungen 2 und 3 kommenden zu spaltenden Gase ($CH_4 + CO_2$) vorgenommen.

Die Austrittstemperatur des Gas-Dampfgemisches ist abhängig von der gesamten Verdichterleistung einer Anlage. Im Prinzip kann die Energie des Gas-Dampfgemisches am Eintritt der Entspannungsturbine bis zur Kondensation des in dem Gas-Dampfgemisch enthaltenen Wasserdampfanteils ausgenutzt werden. In diesem Fall handelt es sich bei der Antriebsturbine um eine sogenannte Kondensationsturbine.

Ist wegen der Mengenverhältnisse der zu verdichtenden Gase nur eine geringere Energiemenge notwendig, so kann die überschüssige Energie für eine zusätzliche Dampferzeugung im Abhitzesystem genutzt werden.

Das verdichtete Synthesegas wird zum einen Teil der Methanol-Synthese in der Katalysereaktionsstufe zugeführt und zum anderen Teil im Kessel 11 verbrannt. Es wird mit reinem Sauerstoff verbrannt. Der

Sauerstoff wird in einer vorgeschalteten Sauerstoff-Anlage gewonnen und dem Synthesegas vor dem Eintritt in den Kessel zugemischt. Aus der Sauerstoff-Anlage wird Stickstoff für beliebige Nutzung abgeführt. Das bei der Methanolsynthese gewonnene Methanol wird als sehr gut verwertbares Produkt aus dem Kreislauf abgezogen.

Das in Figur 8 dargestellte Schema ist eine Beispiel für eine Verfahrensführung, bei der wie bei Figur 7 gearbeitet wird. Das Synthesegas wird aber mit Luft verbrannt. Dabei wird das Abgas aus dem Kessel 11 nicht nur gekühlt, sondern auch noch durch eine erste $CO_2$-Wäsche geführt. Es wird hierbei der aus der Verbrennungsluft stammende Stickstoff aus dieser ersten $CO_2$-Wäsche abgezogen. Nach der ersten $CO_2$-Wäsche wird, wie zuvor erläutert, das Rauchgas mit dem primären Brennstoff Methan vermischt. Das Gemisch wird verdichtet.

Das verdichtete Gemisch wird der Spaltung unterworfen. Das gewonnene Synthesegas-Dampfgemisch wird durch das Abhitzesystem und die zweite $CO_2$-Wäsche geführt. Das verdichtete Synthesegas wird der Methanol-Synthese bzw. unter Zugabe von Luft der Verbrennungsreaktion im Kessel 11 zugeführt. Bei diesem Ausführungsbeispiel wird das $CO_2$-Kreislaufgas zweimal gewaschen.

Das Fließbild in Figur 9 zeigt die Ausführungsform des Verfahrens gemäß Figur 7 für ein Beispiel, bei dem als primärer Brennstoff Methan plus Wasserstoff eingesetzt werden. Dieses Ausführungsbeispiel hat den Vorteil, daß infolge der Zugabe von Wasserstoff viel weniger Methan benötigt wird. Die sonstigen Verfahrensmaßnahmen sind im wesentlichen die gleichen wie bei Figur 7.

Im Fließbild in Figur 10 wird ein in anderer Weise gegenüber der Ausführungsform der Figur 7 abgeändertes Verfahrensbeispiel gezeigt. Wieder wurde Methan als primärer Brennstoff in den Kreislaufprozeß eingeführt. Gleichzeitig werden aber etwa 50 Vol-% des aus der Verbrennungsreaktionsstufe abströmenden Abgases hinter dem Kühler aus dem Kreislaufprozeß in die Atmosphäre abgeblasen. Bei dieser Verfahrensführung sind die erforderlichen Methan- und Dampfmengen anders als bei dem in Figur 7 erläuterten Beispiel.

Das Fließbild in Figur 11 ist ein Ausführungsbeispiel für den Kreislaufprozeß, bei dem ohne Katalysatorreaktionsstufe gefahren wird. Als primärer Brennstoff wird wiederum Methan eingeführt. Dieses wird wie bei Figur 7 beschrieben, mit dem aus der Verbrennungsreaktionsstufe stammenden, gekühlten Rauchgas vermischt.

Das Gemisch wird verdichtet. Anschließend wird das verdichtete Gemisch in der Spaltungsreaktionsstufe zu Synthesegas umgesetzt. Der Energiegehalt des aus der Spaltungsreaktionsstufe austretende Synthesegas-Dampfgemisches wird wie zuvor beschrieben genutzt. Das Synthesegas wird, nach dem es die $CO_2$-Wäsche durchlaufen hat und im Verdichter verdichtet worden ist, mit aus einer Sauerstoff-Anlage gewonnenem Sauerstoff vermischt und dem Kessel zur Verbrennung und Wärmegewinnung zugeführt. Bei diesem Verfahrensbeispiel wird ohne Erzeugung von Methan gearbeitet.

**Patentansprüche**

1. Verfahren zum Erzeugen von Wärmeenergie aus Kohlenwasserstoffen (Primärbrennstoff) durch oxidative Umsetzung in einer Spaltungsreaktionsstufe und einer Verbrennungsreaktionsstufe, wobei primärer Brennstoff und aus der Verbrennungsreaktionsstufe stammendes Rauchgas in der Spaltungsreaktionsstufe zu Synthesegas umgesetzt werden, dadurch gekennzeichnet, daß das Synthesegas aus der Spaltungsreaktionsstufe bzw. eine daraus durch katalytische Umsetzung in einer zusätzlichen Katalysestufe gebildete sauerstoffhaltige Kohlenstoffverbindung als sekundärer Brennstoff mit Sauerstoff in der Verbrennungsreaktionsstufe unter Gewinnung von Wärmeenergie in Form von Dampf oxidativ verbrannt und sämtliches bei der Verbrennung gebildetes Rauchgas zurückgeführt wird und gegebenenfalls für die Bildung von Rauchgas im Kreislauf nicht benötigter sekundärer Brennstoff ausgeschleust wird.

2. Verfahren nach Anspruch 1, dadurch gekennzeichnet, daß das Synthesegas aus der Spaltungsreaktionsstufe zu einem Teil in die Katalysereaktionsstufe und zu einem anderen Teil in die Verbrennungsreaktionsstufe geführt wird.

3. Verfahren nach einem der Ansprüche 1 oder 2, dadurch gekennzeichnet, daß das Synthesegas aus der Spaltungsreaktionsstufe zu einem Teil in die Katalysereaktionsstufe und zu einem anderen Teil einer nebengeschalteten gesonderten Umsetzungsstufe zugeführt wird.

4. Verfahren nach einem der Ansprüche 1 bis 3, dadurch gekennzeichnet, daß in der Spaltungsreaktionsstufe der primäre Brennstoff und das Rauchgas thermisch umgestezt werden.

5. Verfahren nach einem der Ansprüche 1—4, dadurch gekennzeichnet, daß in der Spaltungsreaktionsstufe der primäre Brennstoff und das Rauchgas katalytisch umgesetzt werden.

6. Verfahren nach Anspruch 5, dadurch gekennzeichnet, daß ein nickelhaltiger Katalysator verwendet wird.

7. Verfahren nach Anspruch 5, dadurch gekennzeichnet, daß ein magnesiumhaltiger Katalysator verwendet wird.

8. Verfahren nach einem der Ansprüche 1—7, dadurch gekennzeichnet, daß in der Katalysereaktionsstufe das Synthesegas exotherm in Anwesenheit eines kupferhaltigen Katalysators umgesetzt wird.

9. Verfahren nach einem der Ansprüche 1—8, dadurch gekennzeichnet, daß in der

Verbrennungsreaktionsstufe als Oxidationsmittel reiner Sauerstoff ($O_2$) verwendet wird.

10. Verfahren nach Anspruch 9, dadurch gekennzeichnet, daß Sauerstoff aus einer Luftzerlegung verwendet wird.

11. Verfahren nach einem der Ansprüche 1—8, dadurch gekennzeichnet, daß in der Verbrennungsreaktionsstufe als Oxidationsmittel Luft verwendet, das Abgas aus der Verbrennungsreaktionsstufe in einer Gaszerlegungsstufe in kohlenstoffhaltige und kohlenstofffreie Bestandteile getrennt wird, und die kohlenstofffreien Bestandteile aus dem Kreislauf abgezogen werden.

12. Verfahren nach Anspruch 11, dadurch gekennzeichnet, daß bei der Gaszerlegung die Abtrennung der kohlenstoffhaltigen Bestandteile mittels einer Waschflüssigkeit erfolgt.

13. Verfahren nach Anspruch 11, dadurch gekennzeichnet, daß die Abtrennung der kohlenstofffreien Bestandteile mittels semipermeabler Membran vorgenommen wird.

14. Verfahren nach Anspruch 11, dadurch gekennzeichnet, daß das Abgas aus der Verbrennungsreaktionsstufe in der Gaszerlegungsstufe mittels eines Fremgasdiffusions-Verfahrens zerlegt wird.

15. Verfahren nach einem der Ansprüche 1 bis 14, dadurch gekennzeichnet, daß als primärer Brennstoff Methan oder Erdgas verwendet wird.

16. Verfahren nach Anspruch 15, dadurch gekennzeichnet, daß die Katalysereaktionsstufe als Methanol-Synthese geführt und darin gebildetes, für die Verbrennung in der Verbrennungsreaktionsstufe nicht benötigtes Methanol zur gesonderten Nutzung aus dem Kreislaufprozeß abgezogen und als Produkt gewonnen wird.

**Revendications**

1. Procédé de production d'énergie thermique à partir d'hydrocarbures (combustible primaire) par conversion oxydante dans un étage de réaction de craquage et un étage de réaction de combustion, dans lequel du combustible primaire et du gaz brûlé provenant de l'étage de réaction de combustion sont convertis en gaz de synthèse dans l'étage de réaction de craquage, caractérisé en ce que le gaz de synthèse provenant de l'étage de réaction de craquage ou un composé carboné contenant de l'oxygène qui en est formé par une conversion catalytique dans un étage de catalyse supplémentaire est brûlé par oxydation comme combustible secondaire avec de l'oxygène dans l'étage de réaction de combustion en produisant de l'énergie thermique sous forme de vapeur et tout le gaz brûlé formé lors de la combustion est recylé et le combustible secondaire éventuellement non nécessaire pour la formation du gaz brûlé dans le circuit est collecté.

2. Procédé suivant la revendication 1, caractérisé en ce que le gaz de synthèse provenant de l'étage de réaction de craquage est amené pour partie à l'étage de réaction de catalyse et pour une autre partie à l'étage de réaction de combustion.

3. Procédé suivant la revendication 1 ou 2, caractérisé en ce que le gaz de synthèse provenant de l'étage de réaction de craquage est amené pour partie à l'étage de réaction de catalyse et pour une autre partie à un étage de conversion distinct en aval.

4. Procédé suivant l'une quelconque des revendications 1 à 3, caractérisé en ce que le combustible primaire et le gaz brûlé sont convertis thermiquement dans l'étage de réaction de craquage.

5. Procédé suivant l'une quelconque des revendications 1 à 4, caractérisé en ce que le combustible primaire et le gaz brûlé sont convertis catalytiquement dans l'étage de réaction de craquage.

6. Procédé suivant la revendication 5, caractérisé en ce qu'un catalyseur contenant du nickel est utilisé.

7. Procédé suivant la revendication 5, caractérisé en ce qu'un catalyseur contenant du magnésium est utilisé.

8. Procédé suivant l'une quelconque des revendications 1 à 7, caractérisé en ce que le gaz de synthèse est converti exothermiquement en présence d'un catalyseur contenant dui cuivre dans l'étage de réaction de catalyse.

9. Procédé suivant l'une quelconque des revendications 1 à 8, caractérisé en ce que de l'oxygène pur ($O_2$) est utilisé comme agent d'oxydation dans l'étage de réaction de combustion.

10. Procédé suivant la revendication 9, caractérisé en ce que de l'oxygène provenant d'un fractionnement de l'air est utilisé.

11. Procédé suivant l'une quelconque des revendications 1 à 8, caractérisé en ce que de l'air est utilisé comme agent d'oxydation dans l'étage de réaction de combustion, le gaz brûlé provenant de l'étage de réaction de combustion est séparé dans un étage de fractionnement de gaz en constituants contenant du carbone et constituants exempts de carbone et les constituants exempts de carbone sont soutirés du circuit.

12. Procédé suivant la revendication 11, caractérisé en ce que la séparation des constituants contenant du carbone lors du fractionnement du gaz est exécutée à l'aide d'un liquide de lavage.

13. Procédé suivant la revendication 11, caractérisé en ce que le séparation des constituants exempts de carbone est exécutée au moyen d'une membrane semi-perméable.

14. Procédé suivant la revendication 11, caractérisé en ce que le gaz brûlé provenant de l'étage de réaction de combustion est fractionné dans l'étage de fractionnement de gaz par un procédé de diffusion de gaz d'origine extérieure.

15. Procédé suivant l'une quelconque des revendications 1 à 14, caractérisé en ce que du méthane ou du gaz naturel est utilisé comme combustible primaire.

16. Procédé suivant la revendication 15, caractérisé en ce que l'étage de réaction de catalyse est exploité pour la synthèse du méthanol et le mèthanol qui y est formé et qui n'est pas nécessaire pour la combustion dans l'étage de réaction de combustion est soutiré du circuit pour une utilisation distincte et recueilli comme produit.

**Claims**

1. A process for producing heat energy from hydrocarbons (primary fuel) by oxidative reaction in a cracking reaction stage and a combustion reaction stage, wherein primary fuel and flue gas originating from the combustion reaction stage are reacted in the cracking reaction stage to form synthesis gas, characterised in that the synthesis gas from the cracking reaction stage or an oxygen-bearing carbon compound formed therefrom by catalytic reaction in an additional catalysis stage is subjected to oxidative combustion as a secondary fuel with oxygen in the combustion reaction stage, with the production of heat energy in the form of steam, and all flue gas formed in the combustion operation is recycled and optionally secondary fuel which is not required in the circuit for the formation of flue gas is removed.

2. A process according to claim 1 characterised in that a part of the synthesis gas from the cracking reaction stage is passed into the catalysis reaction stage and another part of the synthesis gas is passed into the combustion reaction stage.

3. A process according to one of claims 1 and 2 characterised in that a part of the synthesis gas from the cracking reaction stage is passed into the catalysis reaction stage and another part of the synthesis gas is passed into a separate reaction stage which is connected in parallel relationship.

4. A process according to one of claims 1 to 3 characterised in that the primary fuel and the flue gas are thermally reacted in the cracking reaction stage.

5. A process according to one of claims 1 to 4 characterised in that the primary fuel and the flue gas are catalytically reacted in the cracking reaction stage.

6. A process according to claim 5 characterised in that a nickel-bearing catalyst is used.

7. A process according to claim 5 characterised in that a magnesium-bearing catalyst is used.

8. A process according to one of claims 1 to 7 characterised in that in the catalysis reaction stage the synthesis gas is exothermally reacted in the presence of a copper-bearing catalyst.

9. A process according to one of claims 1 to 8 characterised in that pure oxygen ($O_2$) is used as an oxidation agent in the combustion reaction stage.

10. A process according to claim 9 characterised in that oxygen from the separation of air is used.

11. A process according to one of claims 1 to 8 characterised in that air is used as the oxidation agent in the combustion reaction stage, the waste gas from the combustion reaction stage is separated in a gas separation stage into carbon-bearing and carbon-free components and the carbon-free components are removed from the circuit.

12. A process according to claim 11 characterised in that in the gas separation operation the carbon-bearing components are separated off by means of a washing liquid.

13. A process according to claim 11 characterised in that the carbon-free components are separated off by means of a semi-permeable membrane.

14. A process according to claim 11 characterised in that the waste gas from the combustion reaction stage is separated in the gas separation stage by means of a foreign gas diffusion process.

15. A process according to one of claims 1 to 14 characterised in that methane or natural gas is used as the primary fuel.

16. A process according to claim 15 characterised in that the catalysis reaction stage is performed as a methanol synthesis operation and methanol which is formed therein and which is not required for combustion in the combustion reaction stage is removed from the cycle for separate use and is obtained as a product.

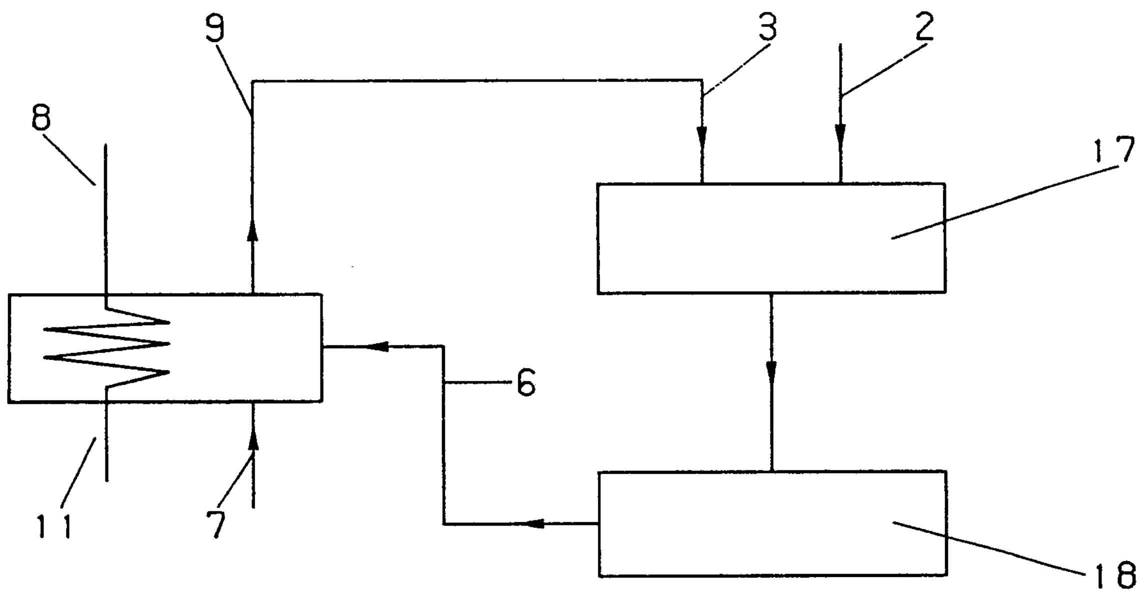


Fig. 1

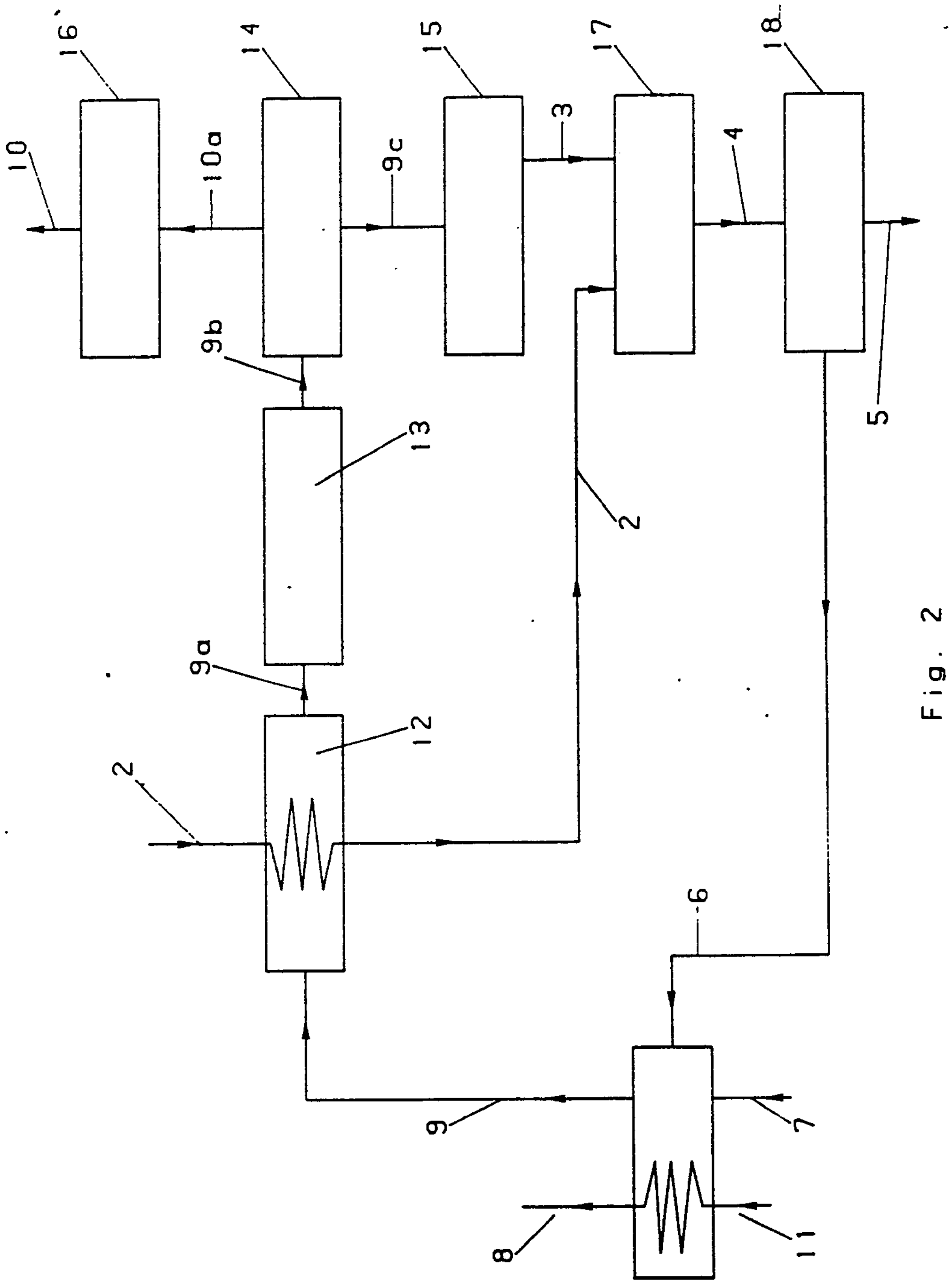


Fig. 2

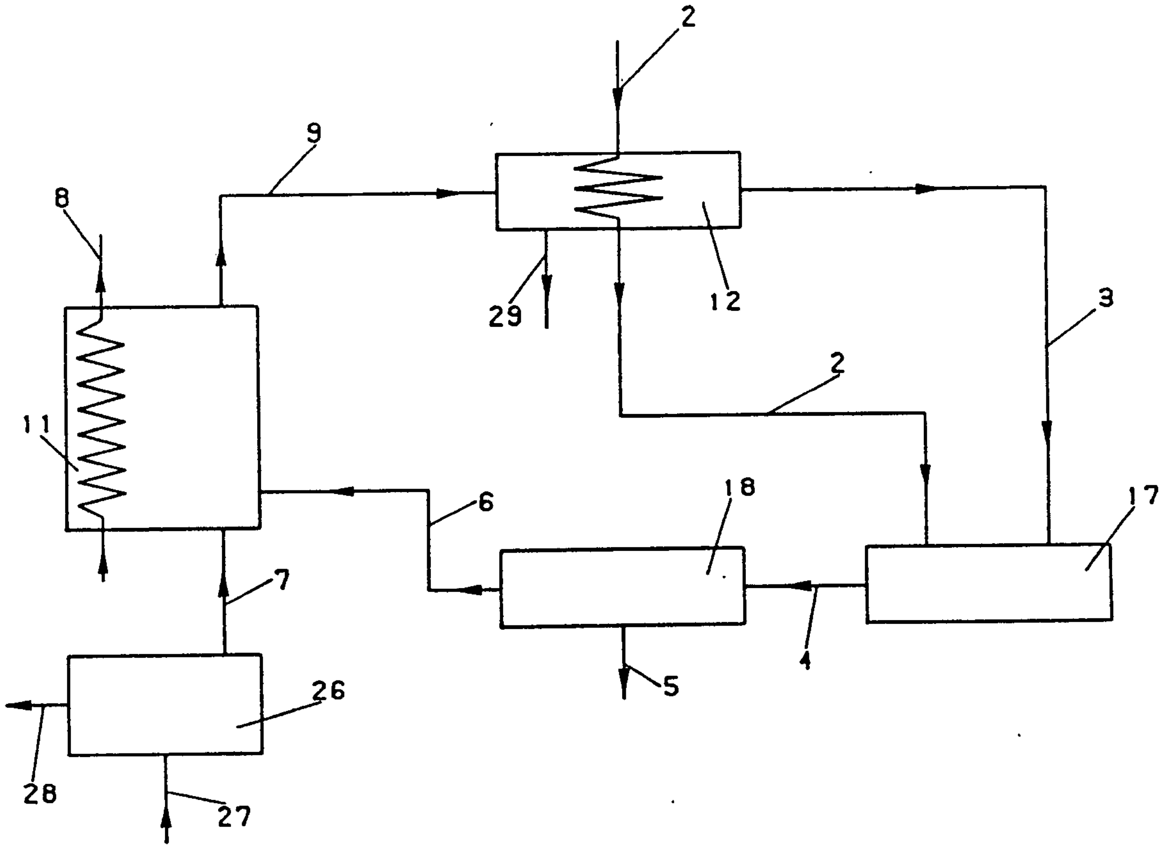


Fig. 3

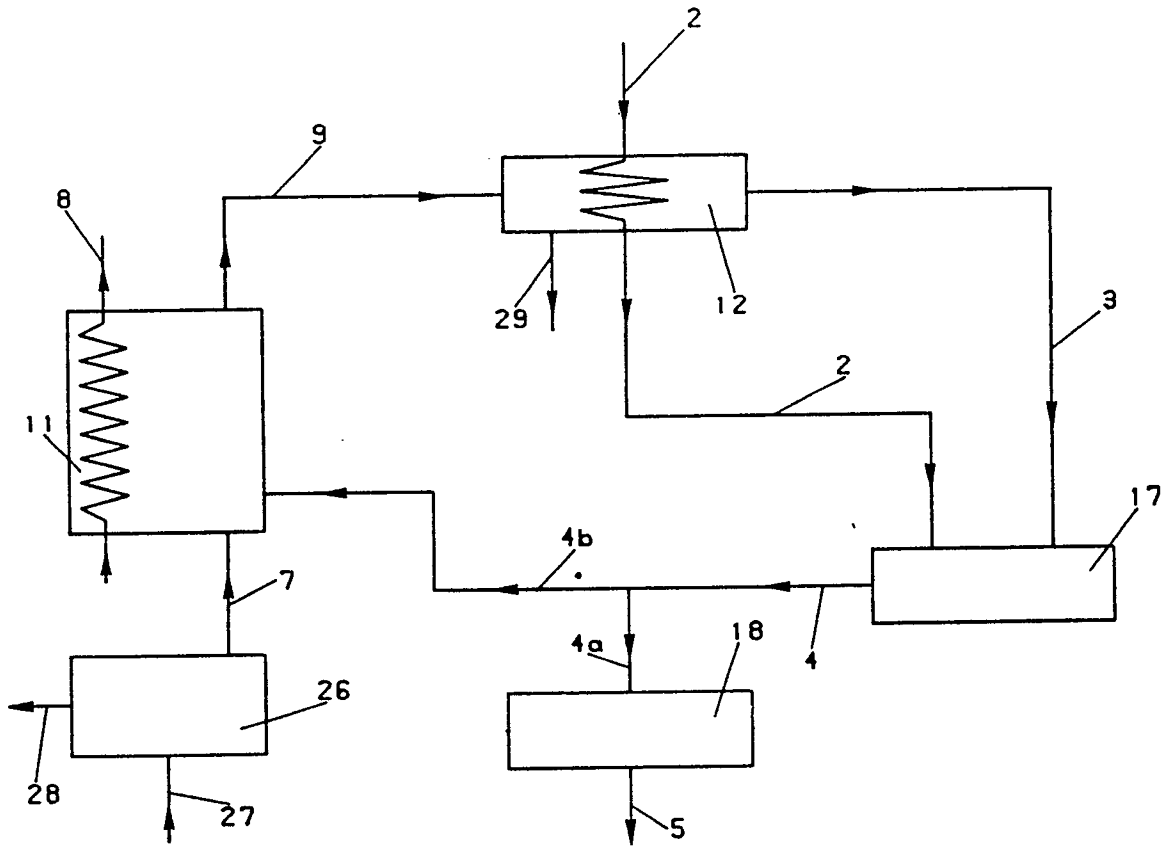


Fig. 4

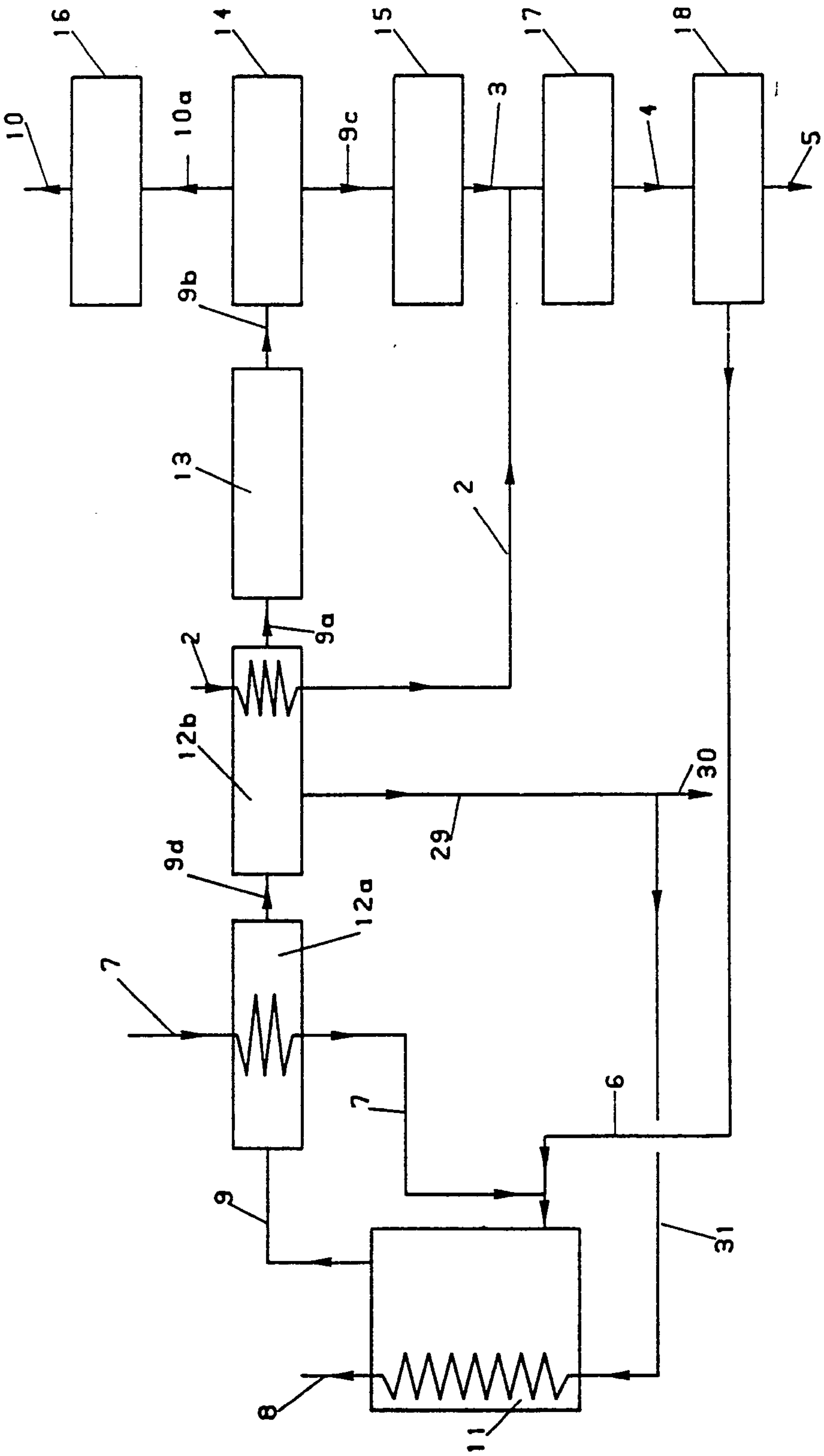


Fig. 5

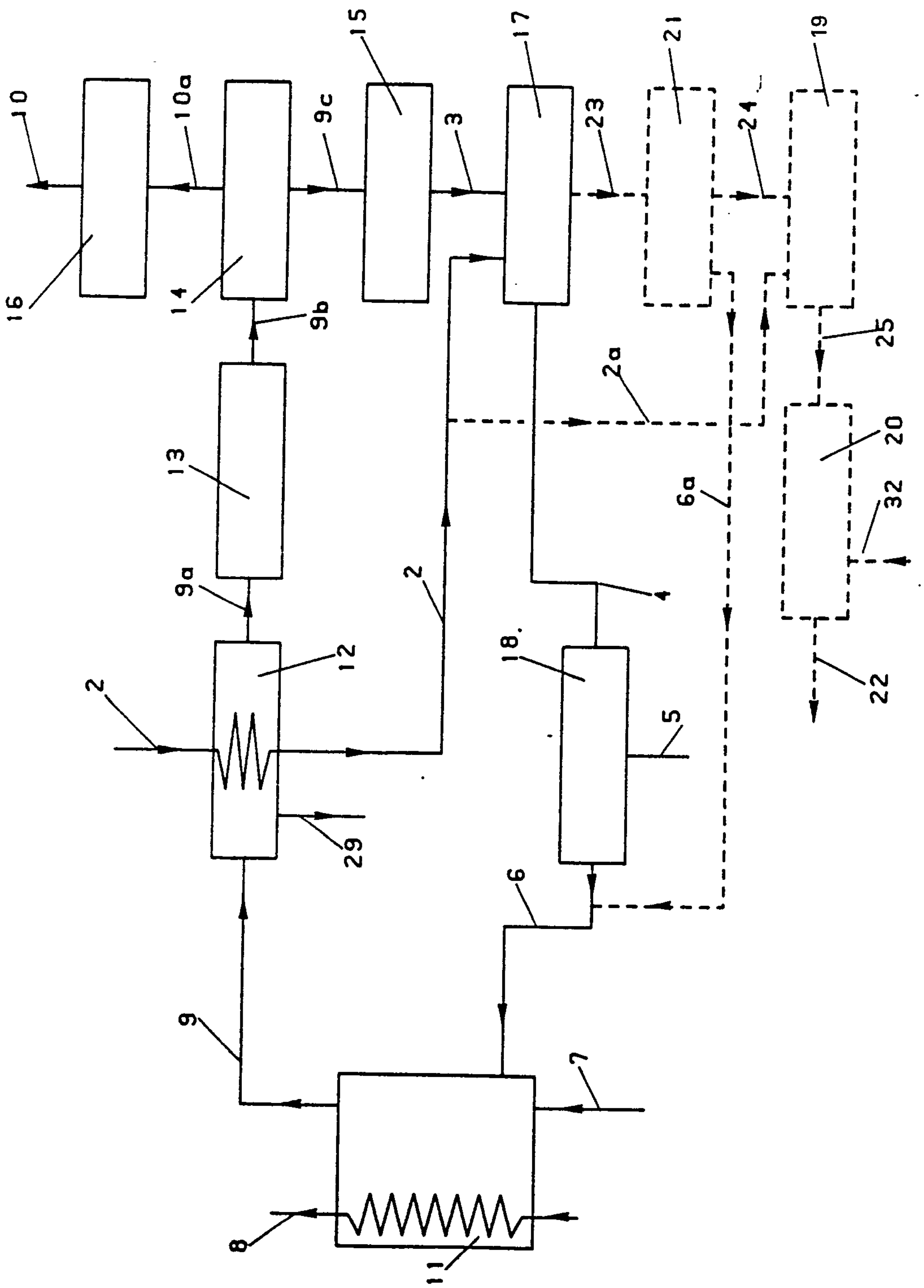

10
10a
9c
15
3
17
23
21
24
19
16
14
9b
2a
25
13
20
6a
32
9a
2
18.
4
12
22
5
29
6
9
7
8
11


Fig. 6

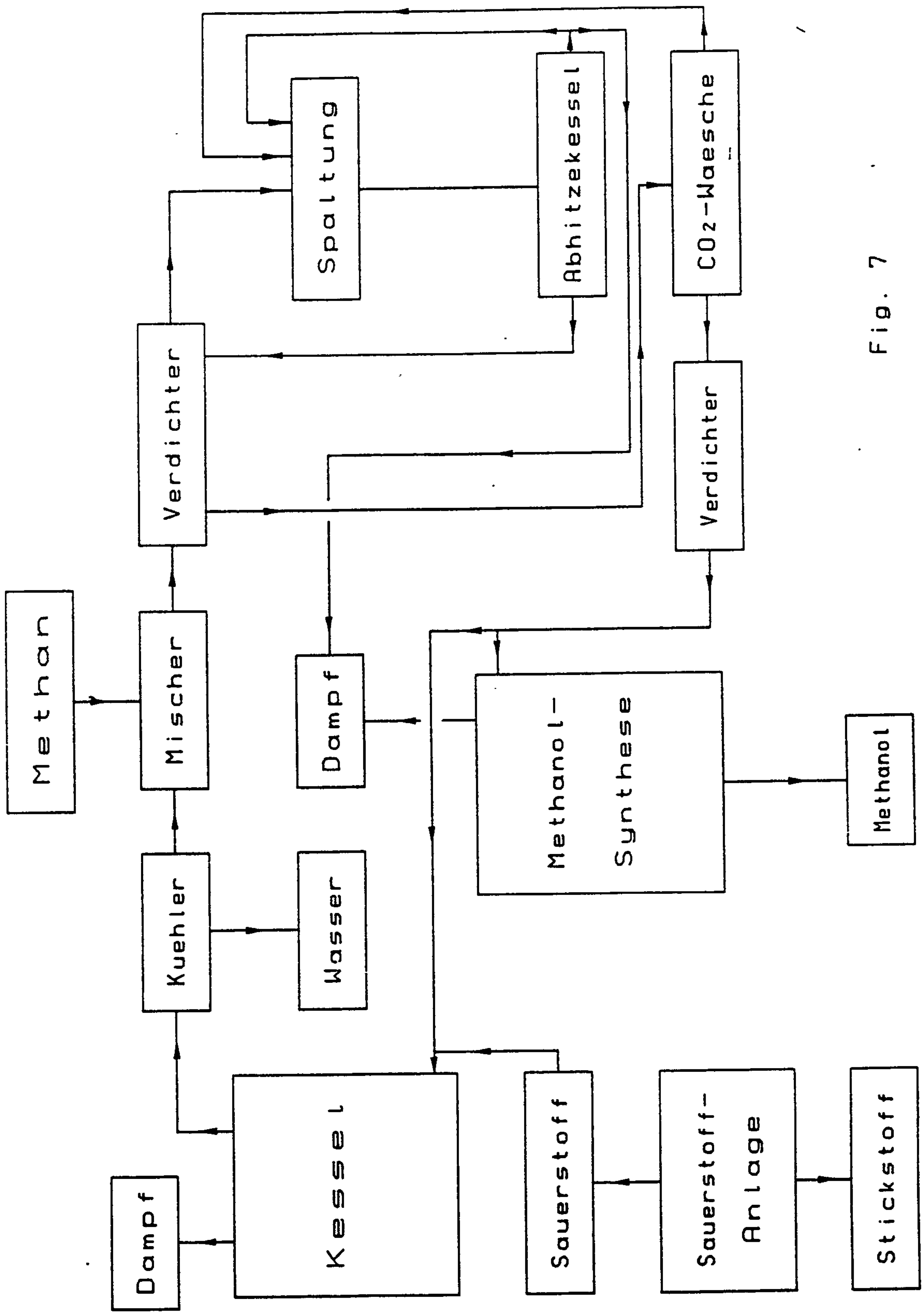
Methan
Mischer
Verdichter
Spaltung
Abhitzekessel
$CO_2$-Waesche
Verdichter
Dampf
Methanol-Synthese
Methanol
Kuehler
Wasser
Kessel
Dampf
Sauerstoff
Sauerstoff-Anlage
Stickstoff

Fig. 7

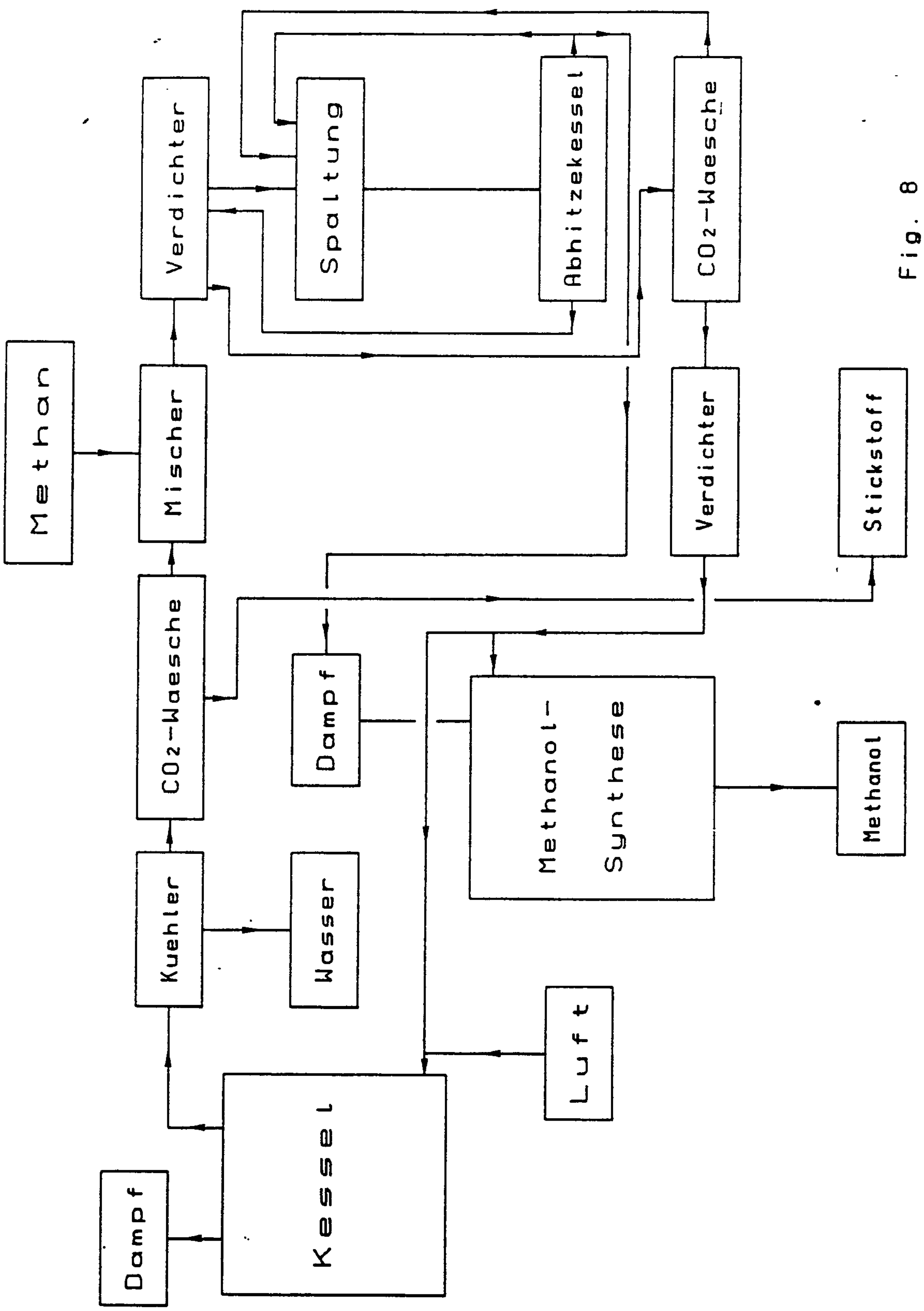
Methan
Mischer
Verdichter
Spaltung
Abhitzekessel
CO2-Waesche
Verdichter
Stickstoff
CO2-Waesche
Dampf
Methanol-Synthese
Methanol
Kuehler
Wasser
Luft
Kessel
Dampf

Fig. 8

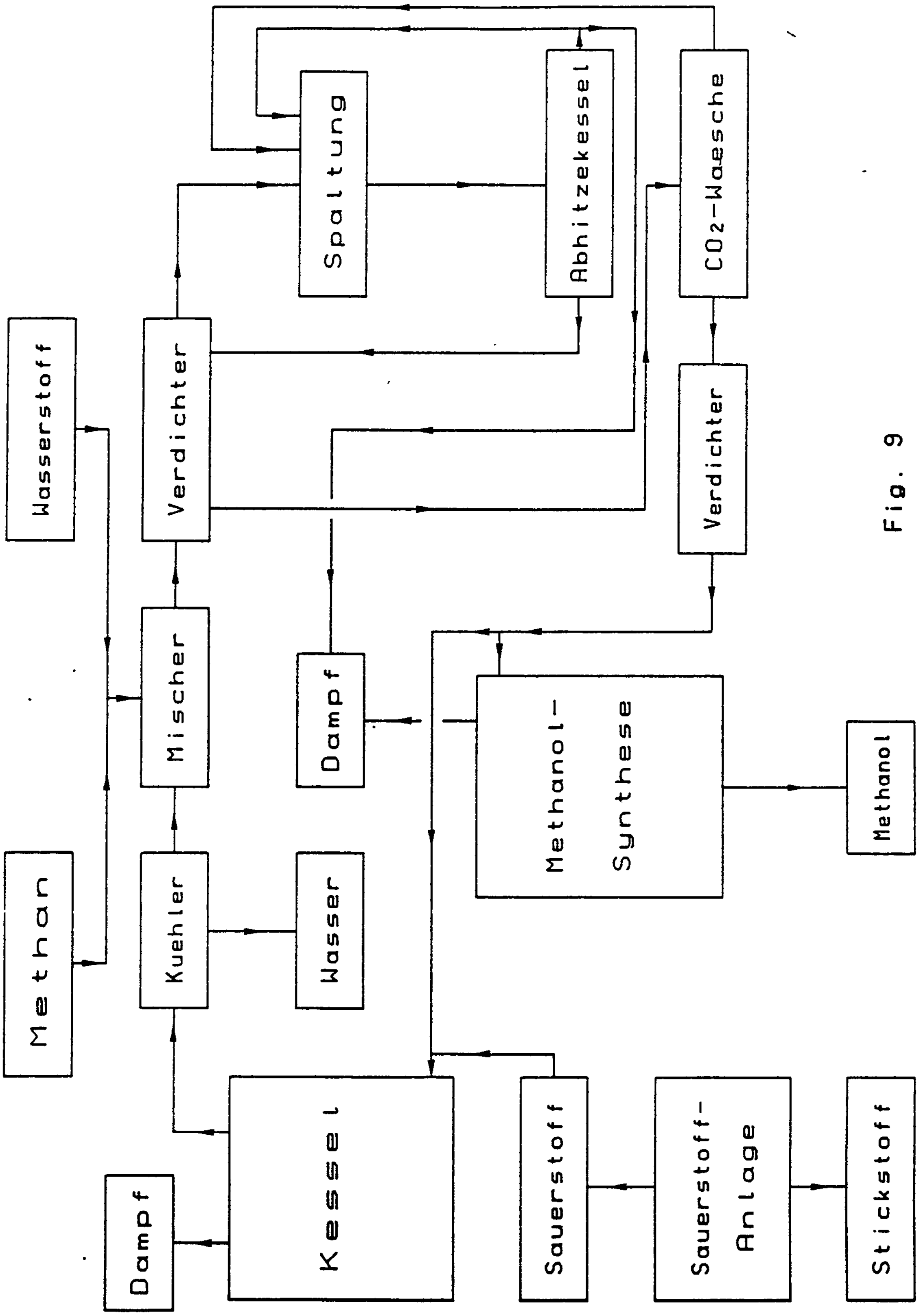
Spaltung
Abhitzekessel
CO₂-Waesche
Wasserstoff
Verdichter
Verdichter
Mischer
Dampf
Methanol-Synthese
Methanol
Methan
Kuehler
Wasser
Kessel
Dampf
Sauerstoff
Sauerstoff-Anlage
Stickstoff

Fig. 9

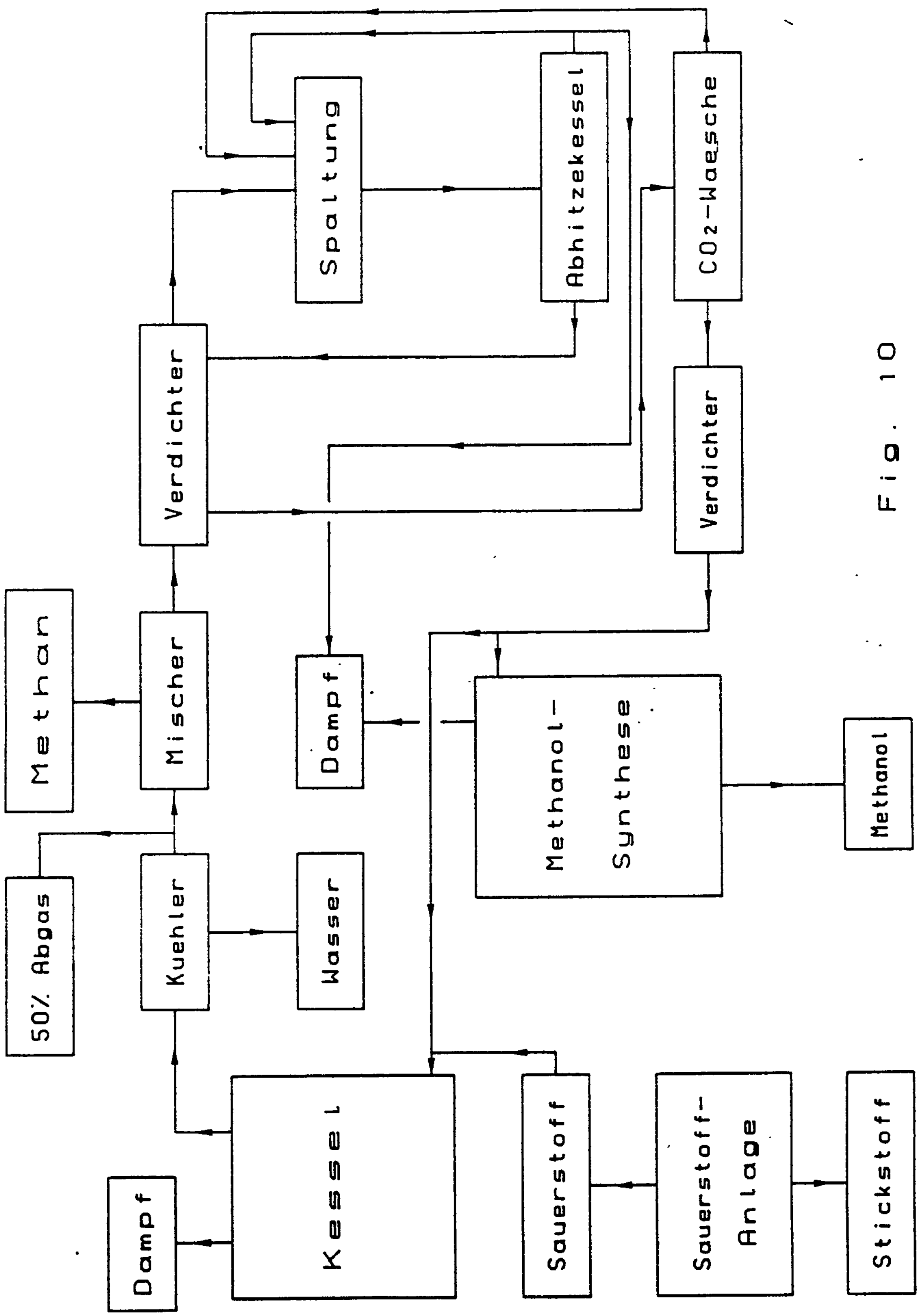
Spaltung
Abhitzekessel
$CO_2$-Waesche
Verdichter
Verdichter
Methan
Mischer
Dampf
Methanol-
Synthese
Methanol
50% Abgas
Kuehler
Wasser
Kessel
Dampf
Sauerstoff
Sauerstoff-
Anlage
Stickstoff

Fig. 10

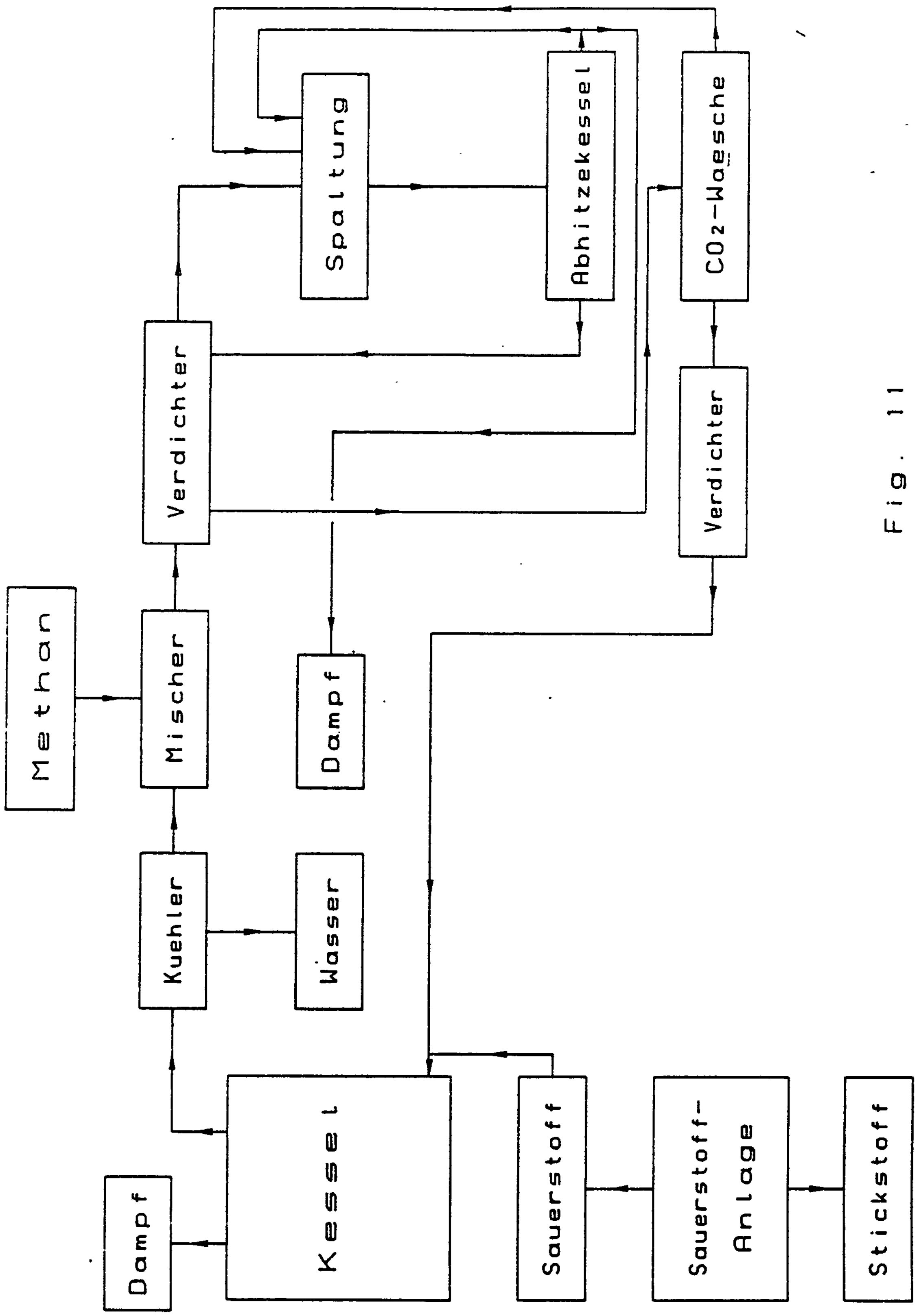
Methan
Mischer
Kuehler
Wasser
Kessel
Dampf
Verdichter
Dampf
Spaltung
Abhitzekessel
CO2-Waesche
Verdichter
Sauerstoff
Sauerstoff-
Anlage
Stickstoff

Fig. 11